# EUROPEAN PATENT SPECIFICATION

(11) **EP 1 900 386 B1**
(45) Date of publication and mention of the grant of the patent: **24.11.2010**
(21) Application number: 06254393.9
(22) Date of filing: 22.08.2006
(51) Int. Cl.: A61M 5/50, A61M 5/32

(54) **Safety seringue**
Sicherheitsspritze
Seringue de sécurité

(43) Date of publication of application: 19.03.2008
(73) Proprietor: Wang, Chih Ming, Keelung City (TW); Tseng, Hsi-Hsun, Keelung City (TW); Lee, Po-Liang, Keelung City (TW); Chuang, Chun-Chieh, Keelung City (TW)
(72) Inventor: Yang, Chung-Yu, Taipei City (TW)
(74) Representative: Carter, Stephen John

(56) References cited:
- EP-A- 1 442 763
- US-A1- 2004 143 215

## Description

### BACKGROUND OF THE INVENTION

### 1. Field of the Invention

The present invention generally relates to a safety syringe, and more particularly, relates to a safety syringe that is capable of retracting a needle unit into a syringe barrel of the safety syringe by low pressure.

### 2. Description of the Prior Art

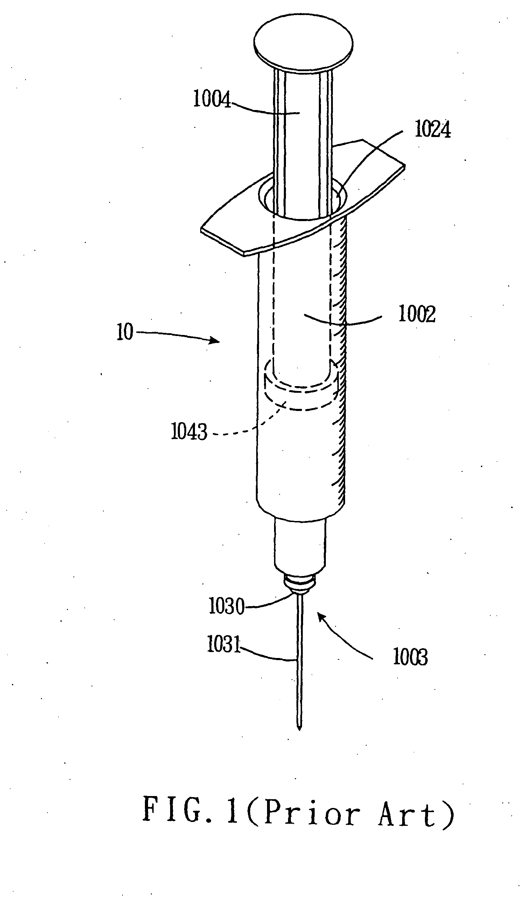

FIG. 1 is a schematic diagram of a conventional syringe. The conventional syringe 10 includes an outer syringe barrel 1002 with scales on its surface and an internal cavity therein for containing a medical solution, a rod 1004 with a flexible rubber-sealing member 1043, and a needle unit 1003. The needle unit 1003 comprises a needle holder 1030 and a needle 1031. An opening (not shown) in a front end of the outer syringe barrel 1002 can be mated with an inner screw thread of the needle holder 1030. A rear end of the needle 1031 is passed through the needle holder 1030 to communicate with an internal cavity of the outer syringe barrel 1002. The rod 1004 can be put into the internal cavity of the outer syringe barrel 1002 via a rear opening 1024 which has a larger cross-sectional area. The outside diameter of the flexible rubber-sealing member 1043 is slightly larger than the inside diameter of the outer syringe barrel 1002 so that the medical solution in the syringe can be flowed out from the needle 1031 while the rod 1004 is pushed from the back end thereof. Similarly, medical solutions can be drawn into the syringe by pulling the rod 1004 backwards.

After injection, the needle unit 1003 is enclosed with a needle sheath by an operating staff manually. Therefore, the operating staff may be stuck by the needle 1031 accidentally, thereby infection possibility is increased. A safety syringe whose needle can be retrieved automatically is required to prevent the operating staff from infections. A hollow inner syringe barrel is generally utilized in the safety syringe to replace the rod of the conventional syringe, so that a needle is automatically retracted into the inner syringe barrel of the safety syringe after injection by way of a mechanism generating an attraction force from the internal cavity of the inner syringe barrel. For example, a needle retraction system of a safety syringe is disclosed in an U.S. Patent No. 5,395,337. A retractable needle medical assembly includes a barrel and a plunger assembly defining an internal passageway within which a retraction member is mounted. The retraction member is biased into the passageway, by means of a spring or a vacuum located within the passageway.

How to automatically retract a needle of a safety syringe is an important issue, relating to efficiency and cost. The U.S patent No. 5, 395, 337 mentioned above provides a solution to retrieve a needle by the spring. However, it is difficult to assemble the spring into an inner syringe barrel having a small volume such as 1 cc or 2 cc. Moreover, a needle unit is generally integrated with the safety syringe, thus the needle unit is not allowed to be replaced. Consequently, a new syringe is needed while the needle of the safety syringe is damaged. Since the needle unit cannot be replaced alone, the cost is increased.

A further example of a safety syringe that is capable of automatically retracting a needle unit into an inner syringe barrel by a low pressure generated from the inner syringe barrel is disclosed in US 2004/0143215 A1. The preamble of claim 1 is based on the disclosure of said document.

### SUMMARY OF THE INVENTION

Preferably, the present invention provides a safety syringe that can overcome the drawbacks of the conventional syringe mentioned above.

Preferably, an object of the present invention is to provide a safety syringe that can resist a low-pressure attraction force from an inner syringe barrel during injection.

This object is obtained by means of the characterising features of claim 1.

Preferably, a further object of the present invention is to provide a safety syringe with a replaceable needle body to reduce waste from changing another whole new syringe while a needle is damaged.

According to an aspect of the present invention there is provided a safety syringe, which comprises an outer syringe barrel, a flexible seat, a needle unit, an inner syringe barrel, a flexible sealing member, and an anti-compressive structure. The outer syringe barrel comprises a front-end opening and a back-end opening. The flexible seat can be fitted tightly against an inner wall of the outer syringe barrel. The needle unit comprises a needle seat and a needle body connecting to the needle seat, wherein the needle seat can be releasably engaged with the flexible seat. The inner syringe barrel comprises a forward end, a backward end, and a hollow body disposed between the forward end and the backward end. An opening located in the forward end of the inner syringe barrel has an inside diameter larger than an outside diameter of the needle seat. The inner syringe barrel can be inserted into the outer syringe barrel through the back-end opening of the outer syringe barrel. The flexible sealing member is locked tightly against an inner wall of the forward end of the inner syringe barrel to seal the opening in the forward end of the inner syringe barrel, and makes the hollow body of the inner syringe barrel a sealed space. The anti-compressive structure can be combined with the flexible sealing member, and used to prevent the flexible sealing member from slipping into the hollow body of the inner syringe barrel. The sealed space within the hollow body of the inner syringe barrel may be in a low-pressure state.

While the inner syringe barrel is moved close to the forward-end opening of the outer syringe barrel, the anti-compressive structure can be combined with the needle seat of the needle unit, the flexible seat is further pushed by the forward end of the inner syringe barrel, and then the flexible seat and the needle seat are separated. In the meanwhile, the flexible sealing member is pushed away from the inner wall of the forward end of the inner syringe barrel by a reaction force from the needle unit, and then retracted into the hollow body of the inner syringe barrel.

### BRIEF DESCRIPTION OF THE DRAWINGS

The foregoing aspects and many of the attendant advantages of this invention will become more readily appreciated as the same becomes better understood by reference to the following detailed description, when taken in conjunction with the accompanying drawings, wherein:
FIG. 1 is a schematic diagram of a conventional syringe;
FIGs. 2A and 2B are cross-sectional views of a safety syringe according to an embodiment of the present invention before and after assembly respectively;
FIGs. 2C to 2F illustrate the relative positions of the components of the safety syringe according to an embodiment of the invention at various stages.
FIGs. 3A to 3E are cross-sectional views of a safety syringe with a replaceable needle body according to another embodiment of the present invention;
FIGs. 4A to 4E are schematic views of a safety syringe with a replaceable needle body according to a further embodiment of the present invention;
FIGs. 5A to 5C illustrate a safety syringe with a replaceable needle body according to yet another embodiment of the present invention; and
FIGs. 6A to 6C are schematic diagrams of a safety syringe with a replaceable needle body according to still yet another embodiment of the present invention.

### DESCRIPTION OF THE PREFERRED EMBODIMENT

Some embodiments and illustrations of the invention will now be described in greater detail. Nevertheless, these illustrations are not scaled or intended to limit the scope of the teachings of the invention, but are merely illustrative. It should be recognized that the present invention can be practiced in a wide range of other embodiments besides those explicitly described, and the scope of the present invention is expressly not limited except as specified in the accompanying claims.

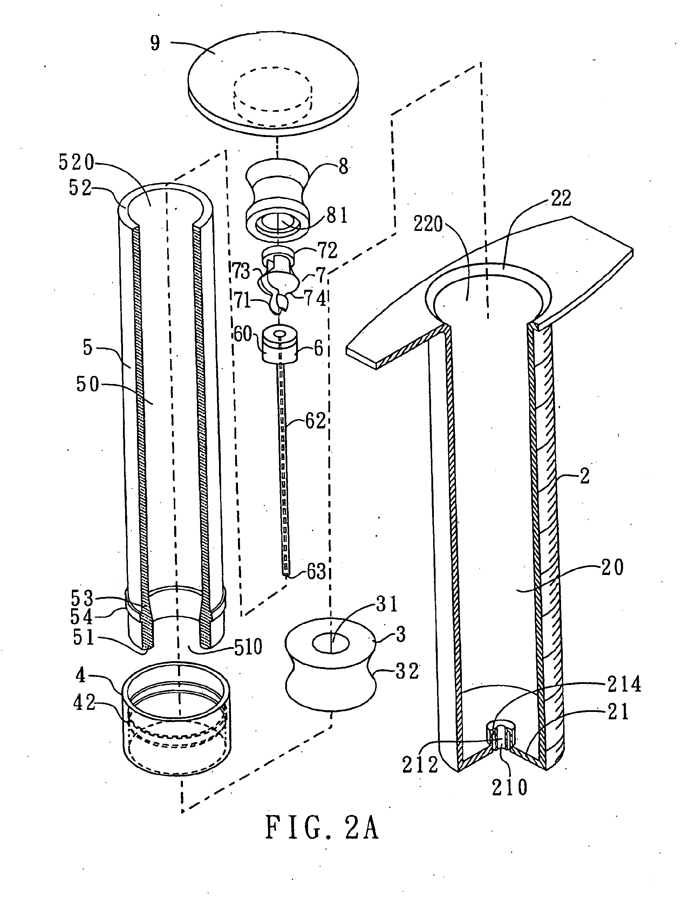

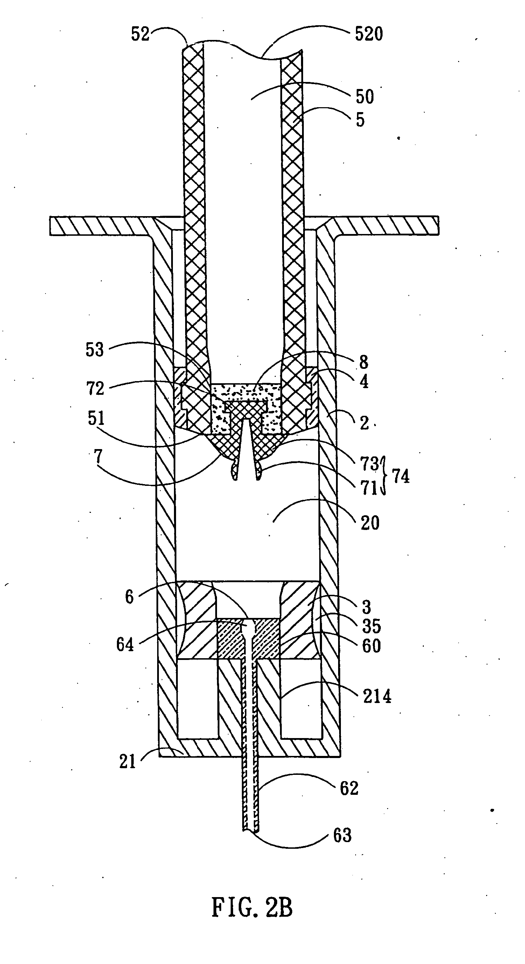

FIGs. 2A and 2B are cross-sectional views of a safety syringe according to an embodiment of the present invention before and after assembly respectively. The safety syringe comprises an outer syringe barrel 2, a flexible seat 3, a needle unit 6, an inner syringe barrel 5, a flexible sealing member 8, and an anti-compressive structure 7. The relations and functions of the above major components, and also the other components in the figures will be illustrated in greater detail below.

In the embodiment, the outer syringe barrel 2 comprises a front end 21, a back end 22, and an elongate hollow body 20 between the front end 21 and the back end 22. The elongate hollow body 20 can be used to contain a liquid, such as a medical solution, and the front end 21 and the back end 22 respectively comprises a front-end opening 210 and a back-end opening 220. The inner syringe barrel 5 can be inserted into the elongate hollow body 20 of the outer syringe barrel 2 through the back-end opening 220. The fluid can be flowed out the safety syringe through the needle unit 6 during injection.

In the embodiment, a combined seat 214 can be disposed adjacent the front end 21 of the outer syringe barrel 2, and the combined seat 214 comprises a through passage 212 communicating with the front-end opening 210 of the outer syringe barrel 2. The combined seat 214 can be integrated with the outer syringe barrel 2, or separately disposed adjacent the front end 21 of the outer syringe barrel 2, the present invention is not limited thereto.

The needle unit 6 comprises a needle seat 60 and a needle body 62 connecting to the needle seat 60. In the embodiment, the needle body 62 is for example a needle. The flexible seat 3 is preferably made of a flexible material. The flexible seat 3 comprises a vertical through hole 31 therein, and an inside diameter of the through hole 31 is slightly smaller than an outside diameter of the needle seat 60, thereby the needle seat 60 of the needle unit 6 can be tightly engaged with the through hole 31 of the flexible seat 3.

In the embodiment, the needle seat 60 engaged with the flexible seat 3 can be disposed on the combined seat 214 inside the elongate hollow body 20 through the back-end opening 220 of the outer syringe barrel 2, the needle body 62 of the needle unit 6 can be passed through the through passage 212 of the combined seat 214 and the front-end opening 210 of the outer syringe barrel 2, and a needle head of the needle body 62 is extended outside the front-end opening 210. The needle seat 60 of needle unit 6 can be held by the combined seat 214, and thus the needle seat 60 is kept a predetermined distance from the front-end opening 210 of the outer syringe barrel 2. The inside diameter of the through hole 31 is preferably equal to or larger than an outside diameter of the combined seat 214. To prevent the medical solution flowing out the outer syringe barrel 20, the flexible seat 3 is preferably fitted tightly against an inner wall of the outer syringe barrel 2. In the embodiment, a concave structure 32 can be formed outside the flexible seat 3, the present invention is not limited thereto. While the flexible seat 3 is fitted tightly against the inner wall of the outer syringe barrel 2, the concave structure 32 of the flexible seat 3 is formed a sealed gap (as shown in FIG. 2B), and the functions of the concave structure 32 will be described below.

The inner syringe barrel 5 is used to replace the rod of the conventional syringe. The inner syringe barrel 5 comprises a forward end S1, a backward end 52, and a hollow body between the forward end 51 and the backward end 52, wherein the forward end 51 comprises a forward-end opening 510. The cross-section area of the inner syringe barrel 5 is smaller than that of the back-end opening 220 of the outer syringe barrel 2, thereby the inner syringe barrel 5 can be inserted into the elongate hollow body 20 of the outer syringe barrel 2 through the back-end opening 220. An inside diameter of the forward-end opening 510 of the inner syringe barrel 5 is larger than an outside diameter of the needle seat such that the needle unit 6 can be retracted into the hollow body 50 of the inner syringe barrel 5.

In the embodiment, the backward end 52 of the inner syringe barrel 5 comprises a backward-end opening 520. In order to make the hollow body 50 of the inner syringe barrel 5 a sealed space afterwards, an end-lid 9 can be provided to seal the backward-end opening 520 of the inner syringe barrel 5. The end-lid 9 can be separately mounted to the backward end 52 of the inner syringe barrel 5 (as shown in FIG. 2A), or integrated with the inner syringe barrel 5, the present invention is not limited thereto.

The flexible sealing member 8 is locked tightly against an inner wall of the forward end 51 of the inner syringe barrel 5 so as to make the hollow body 50 of the inner syringe barrel 5 a sealed space. The inside diameter of the whole hollow body 50 of the inner syringe barrel 5 may not be consistent, for example, in the embodiment, the inside diameter of the forward end 51 of the inner syringe barrel 5 is narrower than that of the hollow body 50 of the inner syringe barrel 5. Thus a convergent part 53 with a smaller inside diameter is formed adjacent to the forward end 51 of the inner syringe barrel 5, and thus the flexible sealing member 8 can be locked tightly against the convergent part 53 of the inner syringe barrel 5.

In the embodiment, the sealed space inside the hollow body 50 of the inner syringe barrel 5 is preferably in a low-pressure state or a vacuum state. While the end-lid 9 or the flexible sealing member 8 is assembled, partial or entire air inside the hollow body 50 can be pumped out to make the sealed space in a low-pressure state or a vacuum state, thereby the needle unit 6 can be retracted into the hollow body 50 of the inner syringe barrel 5 by an attraction force resulted from the low pressure.

To prevent the medical solution overflowing from a gap between the outer syringe barrel 2 and the inner syringe barrel 5, a ring-protruding part 54 or a flexible sealing plunger 4 is preferably disposed outside the forward end 51 of the inner syringe barrel 5 to fit tightly against the inner wall of the outer syringe barrel 2 directly. To ensure that the flexible sealing plunger 4 will not be released from the inner syringe barrel 5, a ring part 42 with a groove formed inside of the flexible sealing plunger 4 can be sleeved around the ring-protruding part 54 located outside the forward end 51 of the inner syringe barrel 5.

The flexible sealing member 8 may slip away from its original position (the convergent part 53) owing to a counterforce resulted from the medical solution during injection, or even the attraction force resulted from the low pressure in the hollow body 50 of the inner syringe barrel 5. Consequently, the anti-compressive structure 7 according to the present invention may prevent the flexible sealing member 8 from slipping into hollow body 50 of the inner syringe barrel 5 during injection. In the embodiment, the anti-compressive structure 7 comprises a head part 72, a neck part 73, and a connection part 71. The head part 72 can be used to combine with the flexible sealing member 8, such that the anti-compressive structure 7 can be moved along with the flexible sealing member 8. The neck part 73 is locked to the forward end 51 of the inner syringe barrel 5 to prevent the flexible sealing member 8 from slipping into the hollow body 50 of the inner syringe barrel 5. The connection part 71 is connected with the neck part 73, and is used to combine with the needle seat 60 of the needle unit 6.

In one embodiment of the invention, the anti-compressive structure 7 can comprise at least a separating part 74 that is constituted of the neck part 73 and the connection part 71. The separating part 74 of the anti-compressive structure 7 is exposed outside the forward end 51 of the inner syringe barrel 5, and the neck part 73 is locked to the forward end 51 of the inner syringe barrel 5. In the embodiment, the anti-compressive structure 7 comprises two individual separating parts 74, and the connection part 71 of anti-compressive structure 7 is exemplified by a protrusion in front of the neck part 73, but the present invention is not limited thereto. The anti-compressive structure 7 can comprise more than two individual separating parts 74, the connection part 71 of the anti-compressive structure 7 may comprise a cave structure or other shapes or designs, or the connection part 71 may be set aside the neck part 72 or somewhere else.

For example, the flexible sealing member 8 can comprise a concave hole 81 to be combined with the head part 72 of the anti-compressive structure, and a width of two stretching sides of the neck part 73 of the separating parts 74 can be slightly larger than the inside diameter of the forward end 51 of the inner syringe barrel 5, thereby the anti-compressive structure 7 is locked to the forward end 51 of the inner syringe barrel 5 (as shown in FIG. 2B). Thus, while the inner syringe barrel 5 is pushed toward the front end 21 of the outer syringe barrel 2, the anti-compressive structure 7 can not only be used to resist the counterforce from the medical solution, but also the attraction force resulted from the low pressure inside the hollow body 50 of the inner syringe barrel 5, such that the flexible sealing member 8 can be maintained in its original position.

The needle seat 60 of the needle unit 6 comprises a combination part to be combined with the connection part 71 of the anti-compressive structure 7. While the anti-compressive structure 7 is combined with the needle unit 6, the width of two stretching sides of the neck part 73 of the separating parts 74 can be reduced to be smaller than the inside diameter of the forward end 51 of the inner syringe barrel 5. In the embodiment, since the connection part 71 of anti-compressive structure 7 is exemplified by a protrusion, the combination part of the needle seat 60 may comprise a cave structure 64, but the present invention is not limited thereto. The combination part of the needle seat 60 can be designed according to the sizes and shapes of the connection part 71 of the anti-compressive structure 7 (or the connection part 71 of the anti-compressive structure 7 can designed according to the combination part of the needle seat 60).

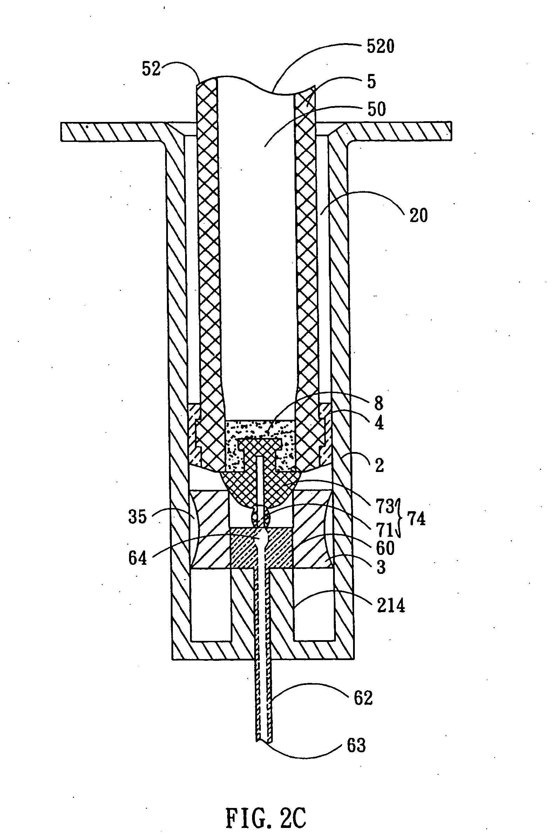

FIGs. 2C to 2F illustrate the relative positions of the components of the safety syringe according to an embodiment of the invention at various stages.

FIG. 2C illustrates the relative positions of the components of the safety syringe according to an embodiment of the invention during injection. In the embodiment, the flexible seat 3 is fitted tightly against the inner wall of the outer syringe barrel 2, and the concave structure 32 of the flexible seat 3 is formed the sealed gap 35, thus the flexible seat 3 is fitted more tightly against the inner wall of the outer syringe barrel 2 by a compressive force from the medical solution so as to prevent the medical solution from overflowing during injection.

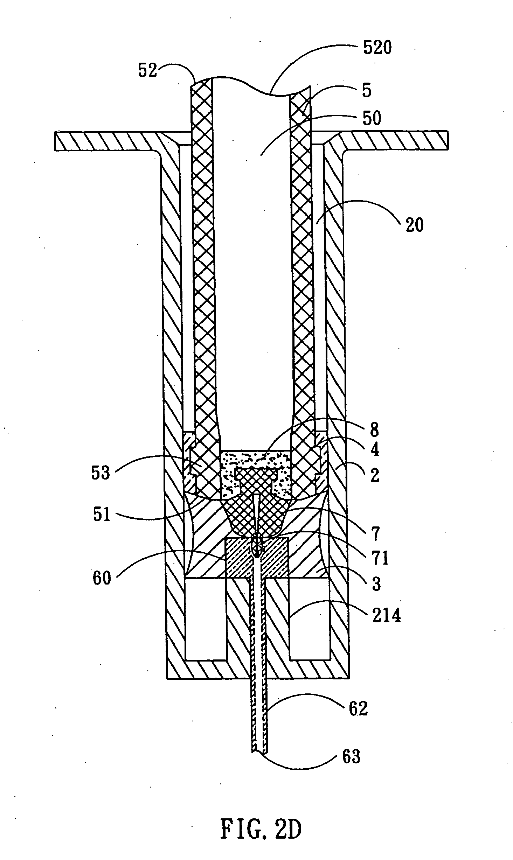

Referring to FIG. 2D, while the inner syringe barrel 5 is further pushed toward the front-end opening 210 of the outer syringe barrel 2 until the injection is completed, the forward-end 51 of the inner syringe barrel 5 is against the flexible seat 3, the two separating parts 74 are drawn to each other, and the connection part 71 of the individual separating parts 74 can be combined with the combination part of the needle seat 60. In the embodiment, the protrusion connection parts 71 are packed into the cave structure 64 of the needle seat 60 to make the anti-compressive structure 7 combine with the needle unit 6 and the width of two stretching sides of the neck parts 73 of the separating parts 74 reduce to be smaller than the inside diameter of the forward end 51 of the inner syringe barrel 5, thus the anti-compressive function of the anti-compressive structure 7 is released. The separating parts 74 of the anti-compressive structure 7 can be drawn inward by a reacting force applied by the needle unit 60 or the flexible seat 3, for example, the needle seat 60 can further comprise a pit in front of the cave structure 64, and a fore end of the individual separating parts 74 of the anti-compressive structure 7 can be drawn inward along a surface of the pit in front of the cave structure 64.

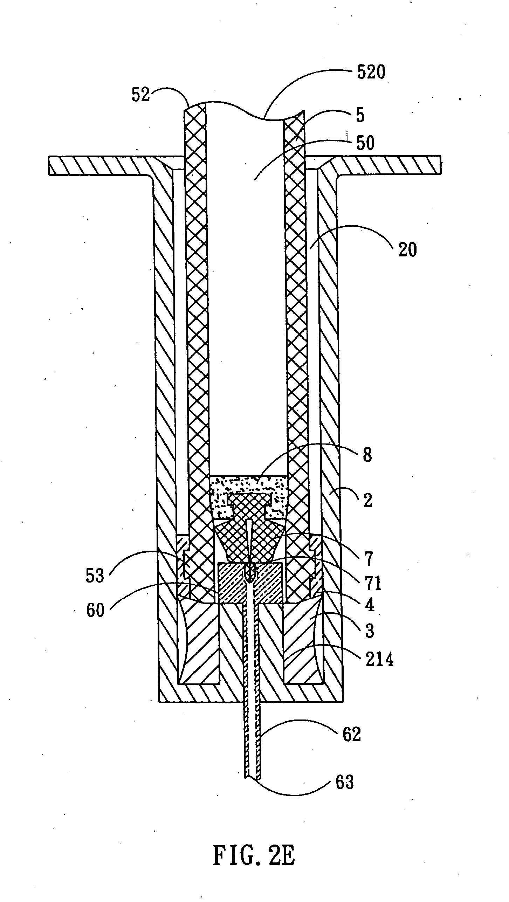

While the inner syringe barrel 5 is pushed even further, since the needle seat 60 of the needle unit 6 is held by the combined seat 214, the needle seat 60 can be kept a predetermined distance from the front-end opening 210 of the outer syringe barrel 2, and the forward end 51 of the inner syringe barrel 5 proceeds to move forward, then the flexible seat 3 is pushed away from the needle seat 60. In the meanwhile, the flexible sealing member 8 is slipped into the hollow body 50 of the inner syringe barrel 5 by the reaction force from the needle seat 60, as shown in FIG. 2E.

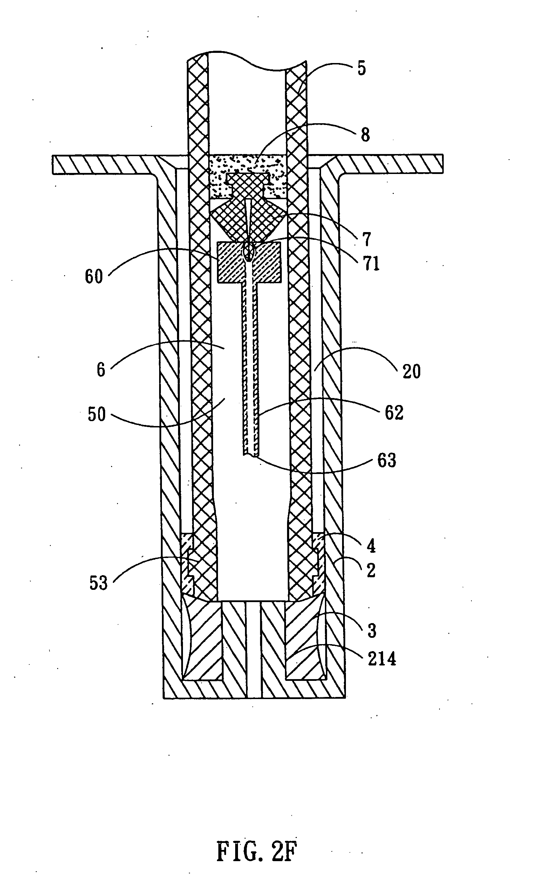

In the embodiment, the hollow body 50 of the inner syringe barrel 5 is in the low-pressure state. While the flexible sealing member 8 and the anti-compressive structure 7 are leaving away from the convergent part 53 and into a more spacious place, a radial compressive force from the inner wall of the hollow body 50 applied to the flexible sealing member 8 is reduced, thus the flexible sealing member 8, the anti-compressive structure 7, and the needle unit 6 combined with the anti-compressive structure 7 are retracted into the hollow body 50 of the inner syringe barrel 5 by the low-pressure attraction force, and the process of automatically retracting the needle unit 6 is done, as shown in FIG. 2F.

The above-mentioned flexible components, such as flexible seat 3, flexible sealing plunger 4, and the flexible sealing member 8, can be made of rubbers or other flexible materials. The anti-compressive structure 7 can be made of resin or plastic, but the present invention is not limited thereto.

According to above FIGs. 2B to 2F, it is appreciated that how the components of the safety syringe according to the present invention are cooperated to retract the needle unit 6 automatically. It is to be understood that the present invention is not limited to the embodiment described above, but various variations of the above-described embodiment may be possible without departing from the scope of the present invention as defined in the appended claims.

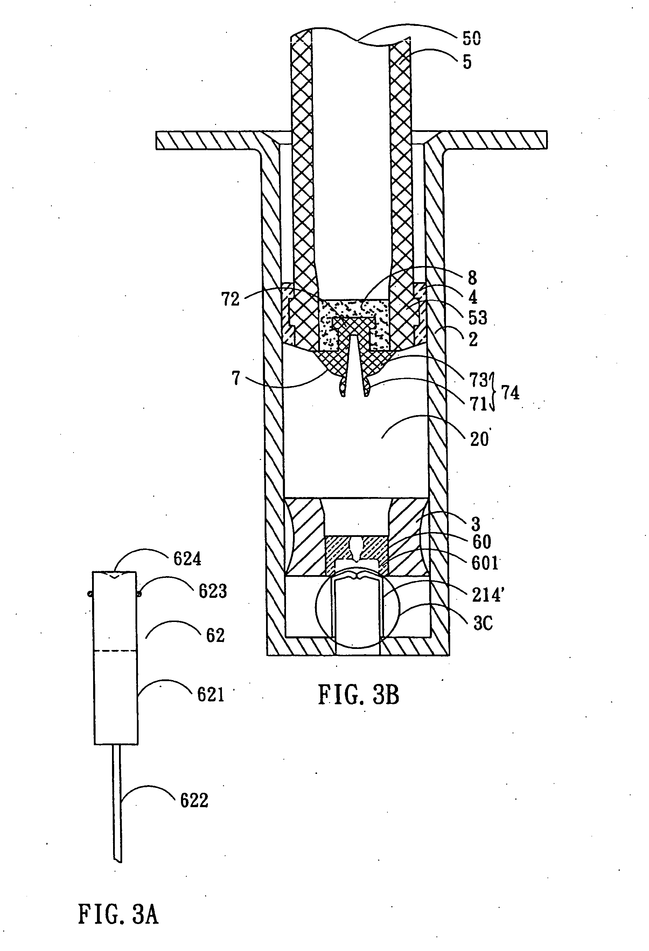

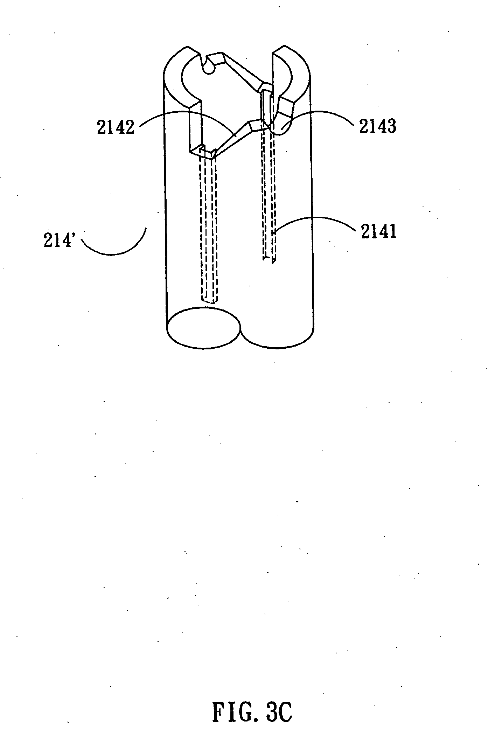

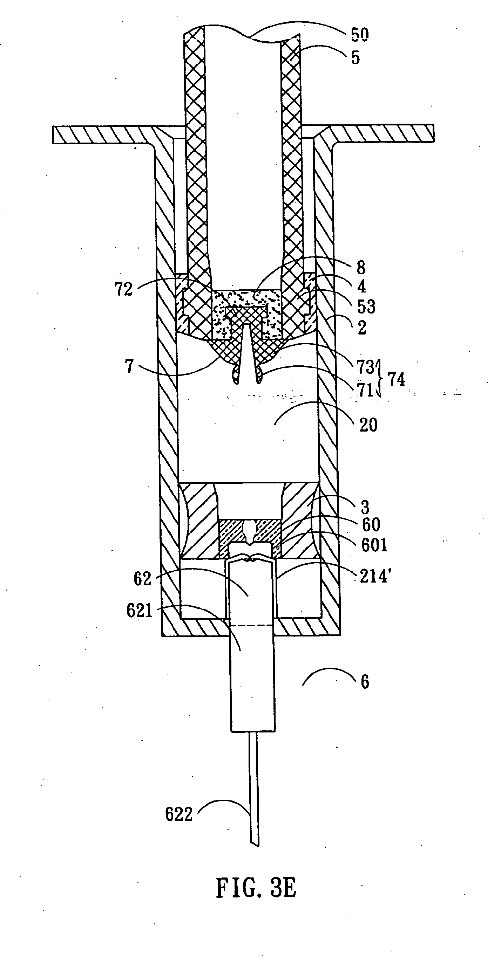

A preferable object of the present invention is to provide a safety syringe with a replaceable needle body to reduce waste from changing another whole new syringe while the needle is damaged. FIGs. 3A to 3E are cross-sectional views of a safety syringe with a replaceable needle body according to another embodiment of the present invention, wherein like reference numerals refer to like parts throughout the various figures unless otherwise specified. FIG. 3C is a cross-sectional view of a safety syringe with a replaceable needle body according to another embodiment of the present invention after assembly, in which the needle seat 60 of the needle unit 6 comprises a joint part 601 to be combined with the needle body 62. In the embodiment, the joint part 601 of the needle seat 60 is exemplified by an inverse U-shaped sealing part, and the needle body 62 comprises a holding part 621 and a needle 622. In this case, the needle body 62 can be separated from the joint part 601 of the needle seat 60.

FIG. 3A illustrates a needle body 62 with a pair of hook structures 623. In the embodiment, the pair of hook structures 623 can be disposed on a sidewall of the holding part 621 of the needle body 62 oppositely, a concave part 624 with a funnel-like shape is on the top of the needle body 62, and the medical solution can be flowed to the needle 622 via the funnel-like shaped concave part 624.

FIG. 3B illustrates a cross-sectional view of the safety syringe with a combined seat 214' having a track 2141, and the track 2141 is located in an inner wall surface of the combined seat 214', and FIG. 3C shows an enlarged sectional view of the combined seat 214' with the track 2141. In the embodiment, a track road 2142 is in the end of the track 2141 along the inner wall surface of the combined seat 214', and a locking part 2143 such as a concave is in the end of the track road 2142. The track 2141, the track road 2142, and the locking part 2143 of the combined seat 214' can be designed corresponding to the hook structures 623 of the needle body 62.

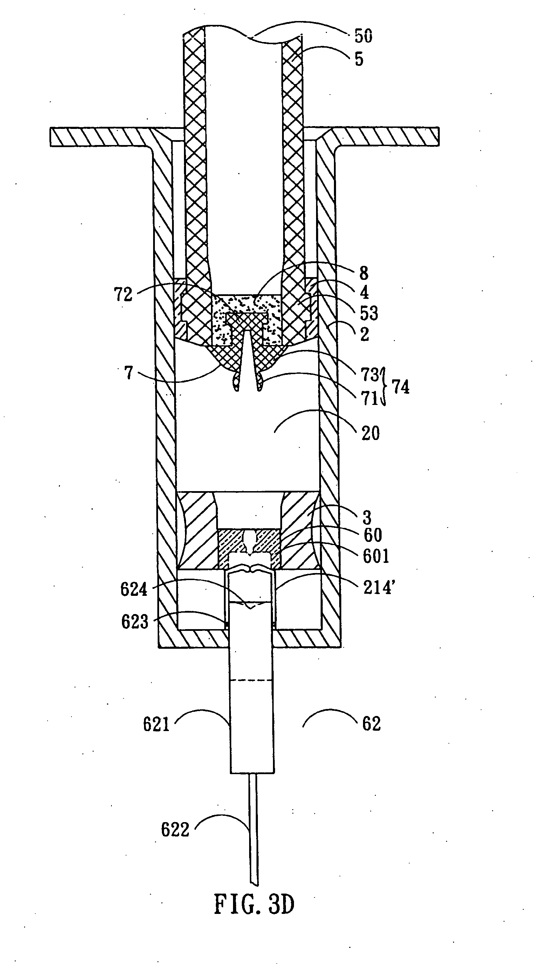

An operator can take the holding part 621 of the needle body 62 and put the hook structures 623 into the combined seat 214' along the track 2141, then the needle body 62 is passed through the through passage 212. Referring to FIGs.3D to 3E, the hook structures 623 of the needle body 62 are moved upward into the safety syringe along the track 2141, and turned along the track road 2142 into the locking part 2143, then a top portion of the needle body 62 is against the joint part 601 of the needle seat 60 tightly, thus the process of combining the needle unit 6 is done. The hook structures 623 of the needle body 62 can be separated from the locking part 2143 of the combined seat 214' by turning the opposite way, thus the needle body 62 is taken off from the joint part 601 of the needle seat 60. In addition, the track road 2142 can comprise a inclined surface.

The joint part 601 of the needle seat 60 can comprise a thread so that the needle body 62 can be screwed to the needle seat 60. It is appreciated that the numbers and shapes of the hook structures 623 are not limited thereto, and the hook structures 623 can be disposed on the outside wall of the needle body 62 symmetrically or asymmetrically. The top portion of the needle body 62 can comprise a planar surface, the present invention is not limited thereto.

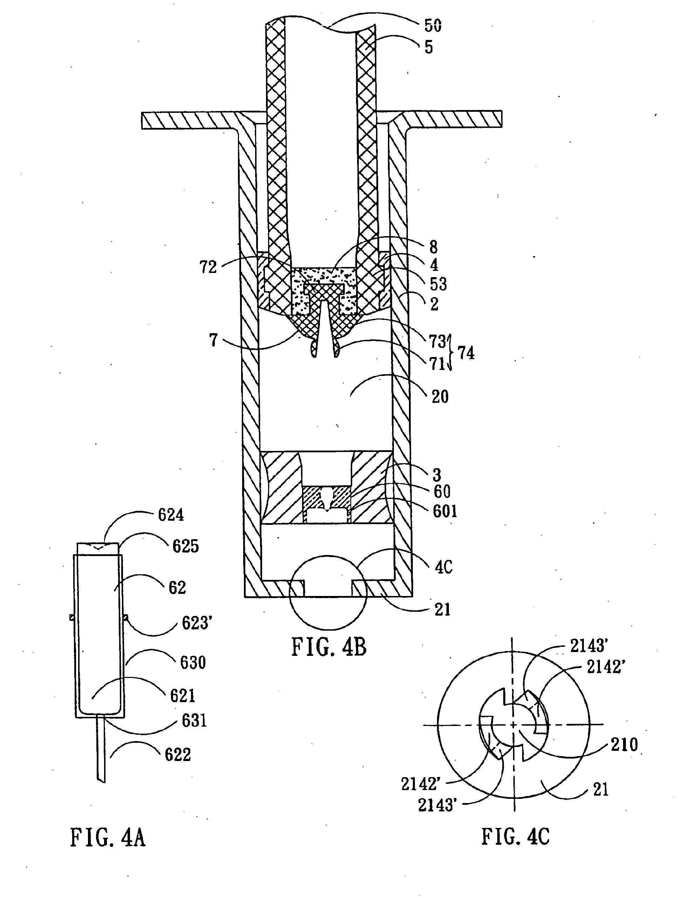

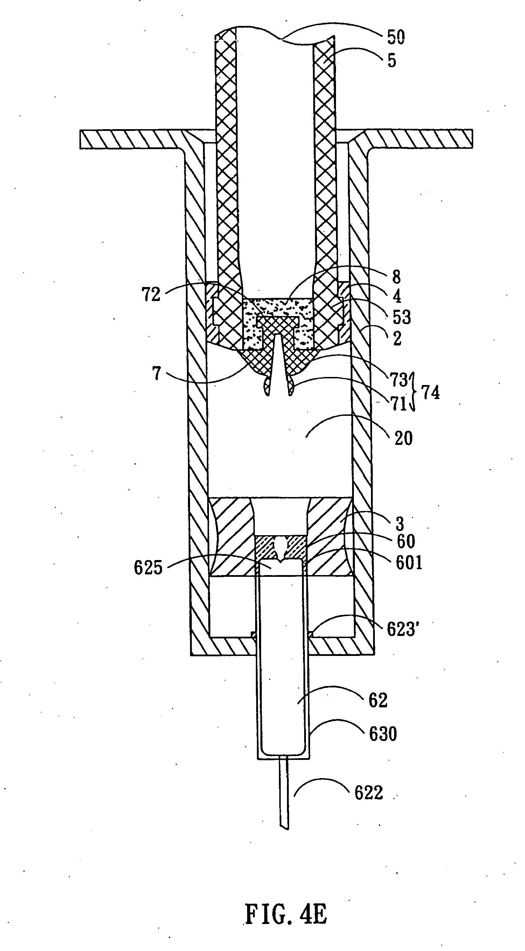

FIGs. 4A to 4E are schematic views of a safety syringe with a replaceable needle body according to a further embodiment of the present invention, wherein like reference numerals refer to like parts throughout the various figures unless otherwise specified. Referring to FIG. 4A, the holding part 621 of the needle body 62 is disposed within a needle sheath 630, and the needle 622 is passed through a hole 631 in a front-end wall of the needle sheath 630 to be exposed outside of the needle sheath 630. In the embodiment, a pair of hook structures 623' can be disposed on an outside wall of the needle sheath 630, and the needle body 62 can be separated from the needle sheath 630.

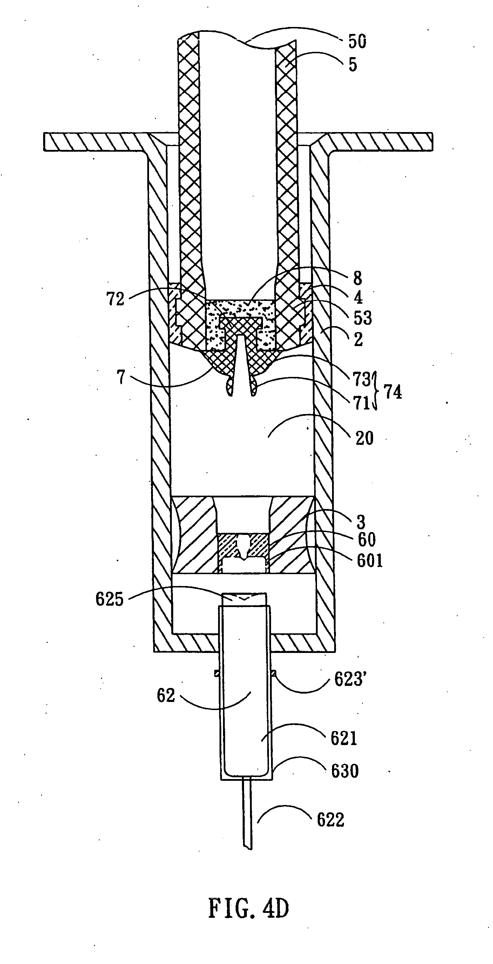

In the embodiment, there is not a combined seat disposed adjacent to the front end 21 of the outer syringe barrel 2. The needle sheath 630 with hook structures 623' can be locked to the front end 21 of the outer syringe barrel 2 by the similar way as illustrated in FIG. 3C, and FIG. 4C shows a vertical sectional view of the front end 21 of the outer syringe barrel 2. Referring to FIGs. 4D to 4E, the operator can take the needle sheath 630 and put the needle body 62 into the safety syringe, the hook structures 623' of the needle sheath 630 are turned along the track road 2142' into the locking part 2143' to hold the needle sheath 630. In the meantime, a top portion of the needle sheath 630 is against a bottom of the needle seat 60 tightly, and the top portion 625 of the needle body 62 can be combined with the joint part 601 of the needle seat 60.

In the embodiment, the needle seat 60 is held by the needle sheath 630, and thus the needle seat 60 can be kept a predetermined distance from the front-end opening 210 of the outer syringe barrel 2. The needle sheath 630 can be taken off by turning the opposite way, thus the needle sheath 630 can be reused.

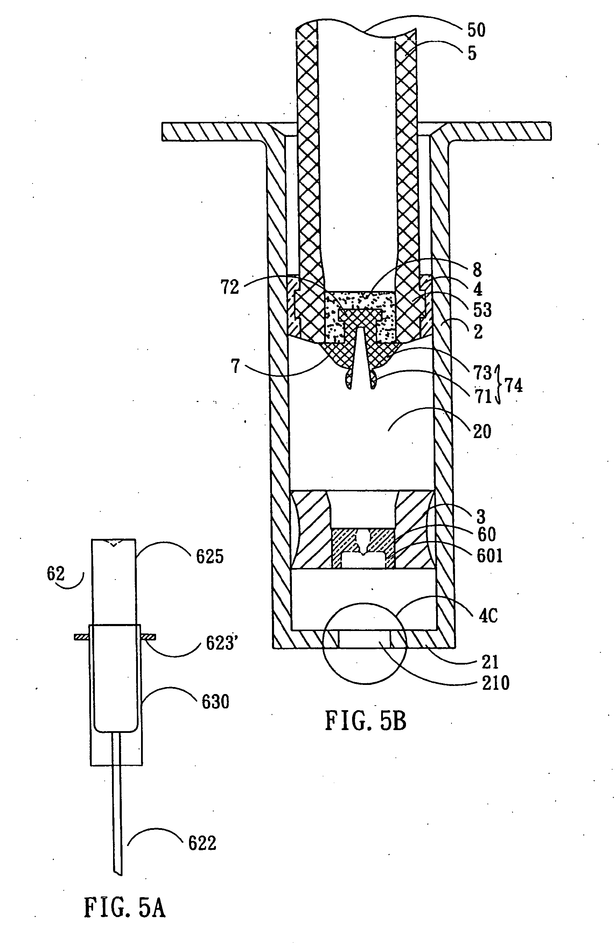

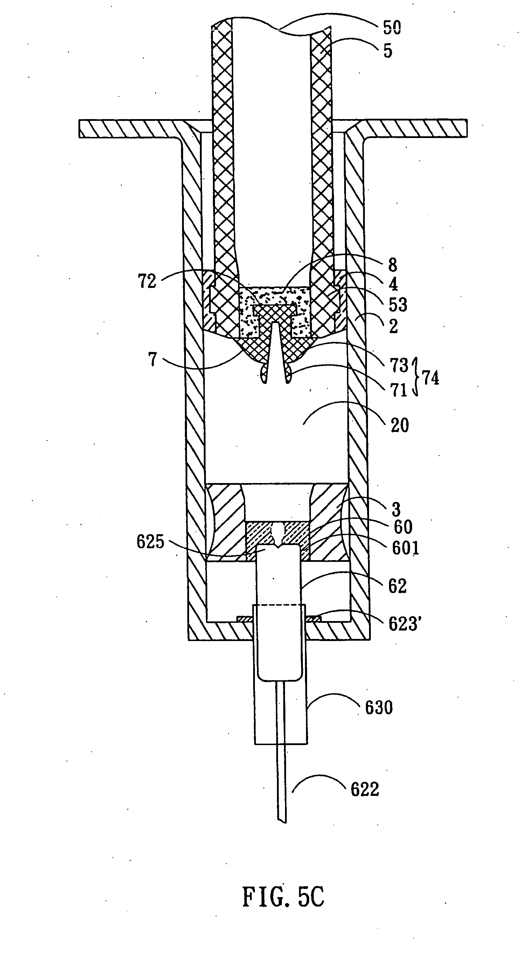

FIGs. 5A to 5C illustrate a safety syringe with a replaceable needle body according to yet another embodiment of the present invention. The length of the needle sheath 630' above the hook structures 623' is shorter than that as illustrated in FIGs. 4A to 4E, and most of the top portion of the needle body 62 is exposed outside of the needle sheath 630'. In the embodiment, the way to lock the hook structures 623' (with reference to FIG. 4C), the way to combine the needle body 62 with the joint part 601 of the needle seat 60, and the way to retract the needle unit 6, are the same as those illustrated above. The sizes or the numbers of the hook structures 623' can be changed according to the needs and requirements, the present invention is not limited thereto.

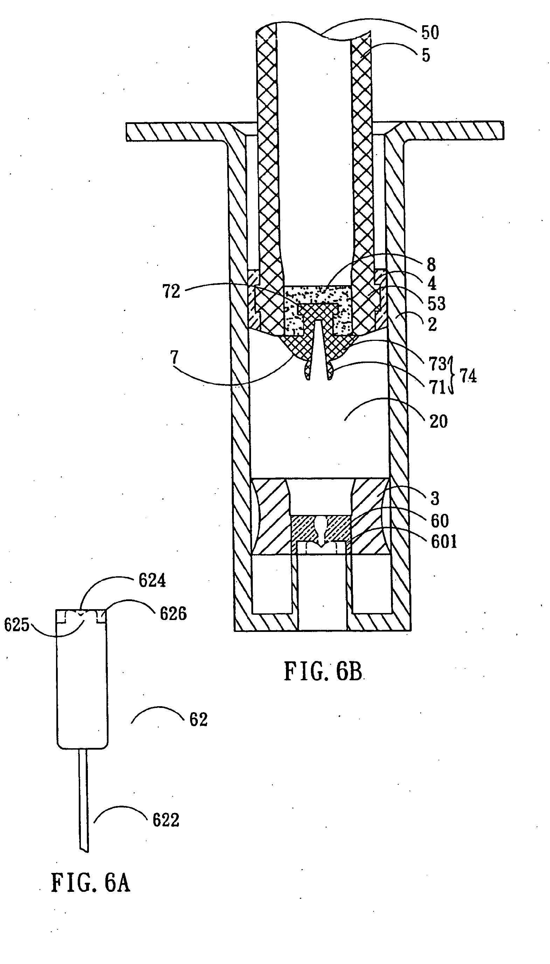

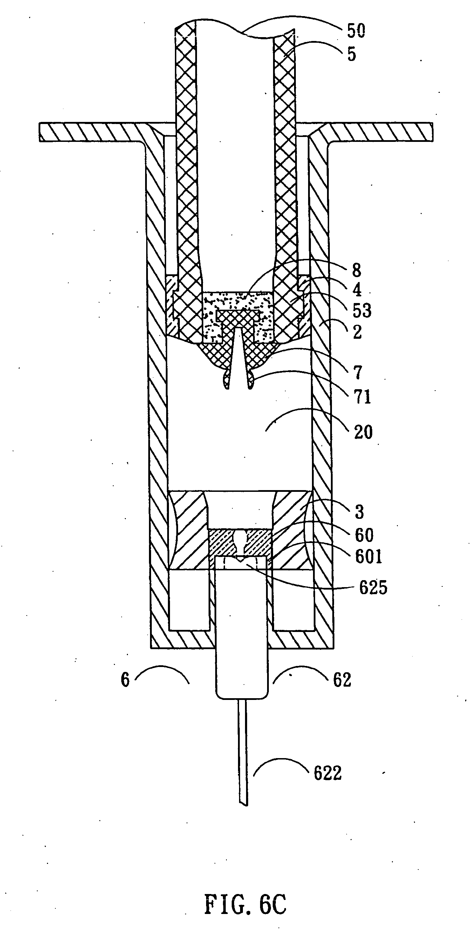

FIGs. 6A to 6C are schematic diagrams of a safety syringe with a replaceable needle body according to still yet another embodiment of the present invention, wherein like reference numerals refer to like parts throughout the various figures unless otherwise specified. Referring to FIG. 6A, the top portion 625 of the needle body 62 comprises a thread 626 and a funnel-like shaped concave part 624, and the bottom of the needle body 62 comprises a needle 622. Referring to FIG. 6B, a joint part 601 of the needle seat 60 comprises a thread that can be mated with the thread 626 in the top portion 625 of the needle body 62, such that the needle body 62 can be screwed to the joint part 601 of the needle seat 60. In the embodiment, the needle body 62 is combined with the needle seat 60 to assemble a needle unit 6 by way of screwing, thus the needle unit 6 can be retracted into the hollow body 50 of the inner syringe barrel 5 along with the flexible sealing member 8, as shown in FIG. 6C.

Several designs of needle units with replaceable needle bodies and corresponding safety syringes are provided above to illustrate how a needle seat is combined with a replaceable needle body to assemble a needle unit, and how the needle unit is retracted into the hollow body of the inner syringe barrel by the low-pressure attraction mechanism according to the present invention.

Compared to the conventional syringe, the safety syringes of the present invention may be used to prevent an operating staff from infections effectively. Compared to the U.S. patent 5,395,337, the safety syringes of the present invention may be more environment friendly because the needle unit may be retracted by a low pressure, not other components (such as spring). In addition, the needle body of the needle syringe according to the present invention may be replaced while the needle is damaged, thus the safety syringe is not necessary to be changed entirely. The safety syringes provided by the present invention may not only be economic but may also be environment friendly.

Although specific embodiments have been illustrated and described, it will be obvious to those skilled in the art that various modifications may be made without departing from what is intended to be limited solely by the appended claims.

## Claims

1. A safety syringe, comprising:
an outer syringe barrel (2) having a front-end opening (210) and a back-end opening (220);
a flexible seat (3) fitted to an inner wall of said outer syringe barrel (2);
a needle unit (6) comprising a needle seat (60) and a needle body (62) connecting to said needle seat (60), wherein said needle seat (60) is releaseably engaged with said flexible seat (3);
an inner syringe barrel (5) having a forward end (51), a backward end (52), and a hollow body (50) between said forward end (51) and said backward end (52), wherein said forward end (51) of said inner syringe barrel (5) comprises a forward-end opening (510), an inside diameter of said forward-end opening (510) is larger than an outside diameter of said needle seat (60), and said inner syringe barrel (5) can be inserted into said outer syringe barrel (2) through said back-end opening (220) of said outer syringe barrel (2);
a flexible sealing member (8) locked against an inner wall of said forward end (51) of said inner syringe barrel (5) to make said hollow body (50) of said inner syringe barrel (5) a sealed space; **characterized by**:
an anti-compressive structure (7) combined with said flexible sealing member (8) to prevent said flexible sealing member (8) from slipping into said hollow body (50) of said inner syringe barrel (5) during injection;
whereby while said inner syringe barrel (5) is moved toward said front-end opening (210) of said outer syringe barrel (2), said anti-compressive structure (7) is combined with said needle unit (6), said front end (51) of said inner syringe barrel (5) is against said flexible seat (3) to release the combination of said needle unit (6) and said flexible seat (3), and then said flexible sealing member (8) is pushed into said hollow body (50) of said inner syringe barrel (5) by a reaction force applied from said needle unit (6).

2. The safety syringe according to claim 1, wherein said sealed space within said hollow body (50) of said inner syringe barrel (5) is in a low-pressure state.

3. The safety syringe according to claim 1 or 2, wherein said anti-compressive structure (7) comprises:
a head part (72) capable of combining with said flexible sealing member (8);
a neck part (73) connecting with said head part (72), said neck part (73) is locked to said forward end (51) of said inner syringe barrel (5) to prevent said flexible sealing member (8) from slipping into said hollow body (50) of said inner syringe barrel (5); and
a connection part (71) connecting with said neck part (73), said connection part (71) is capable of combining with said needle seat (60) of said needle unit (6).

4. The safety syringe according to claim 3, wherein anti-compressive structure (7) comprises at least a separating part (74) constituted of said neck part (73) and said connection part (71).

5. The safety syringe according to claim 4, wherein said needle seat (60) of said needle unit (6) comprises a combination part, while said inner syringe barrel (5) is moved toward said front-end opening (210) of said outer syringe barrel (2), said separating part (74) of said anti-compressive structure (7) is drawn inward and an outside edge of said neck part (73) of said anti-compressive structure (7) is reduced within said forward-end opening (510) of said inner syringe barrel (5), said connection part (71) of said separating part (74) is combined with said combination part of said needle unit (6), thereby said anti-compressive structure (7) is combined with said needle unit (6).

6. The safety syringe according to claim 1 or 2, wherein said needle seat (60) of said needle unit (6) comprises a joint part (601) to be combined with said needle body (62), and said needle body (62) can be separated from said joint part (601) of said needle seat (60).

7. The safety syringe according to claim 1 or 2, further comprising a combined seat (214') disposed adjacent a front end (21) of said outer syringe barrel (2), said combined seat (214') comprising a through passage communicating with said front-end opening (210) of said outer syringe barrel (2), said needle seat (60) engaged with said flexible seat (3) is held by said combined seat, thereby said needle seat (60) is kept a predetermined distance from said front-end opening (210) of said outer syringe barrel (2), and said needle body (62) is passed through said through passage of said combined seat and said front-end opening (210) of said outer syringe barrel (2).

8. The safety syringe according to claim 6, further comprising a combined seat disposed adjacent a front end (21) of said outer syringe barrel (2), said combined seat (214') comprising a through passage communicating with said front-end opening (210) of said outer syringe barrel (2), said needle seat (60) engaged with said flexible seat (3) is held by said combined seat (214'), thereby said needle seat (60) is kept a predetermined distance from said front-end opening (210) of said outer syringe barrel (2), and said needle body (62) is passed through said through passage of said combined seat (214') and combined with said joint part (601) of said needle seat (60).

9. The safety syringe according to claim 8, wherein said combined seat (214') comprises an inner wall surface, a track (2141) is located in said inner wall surface of said combined seat (214'), a track road (2142) is in the end of said track (2141) along said inner wall surface of said combined seat (214'), and a locking part (2143) is in the end of said track road (2142).

10. The safety syringe according to claim 9, wherein said needle body (62) comprises a hook structure (623) corresponding to said track (2741), said needle body (62) is passed through said through passage (212) along said track (2141), and said hook structure (623) of said needle body (62) is locked with said locking part (2143) along said track road (2142) thus said needle body (62) is combined with said joint part (601) of said needle seat (60).

## Patentansprüche

1. Sicherheitsspritze, umfassend:
einen äußeren Spritzenzylinder (2) mit einer vorderen Öffnung (210) und einer hinteren Öffnung (220);
einen flexiblen Einsatz (3), der an der Innenwand des äußeren Spritzenzylinders (2) eingepasst ist;
eine Nadeleinheit (6), die einen Nadelsitz (60) und einen Nadelkörper (62), der mit diesem Nadelsitz (60) verbunden ist, umfasst, wobei der Nadelsitz (60) in lösbarem Eingriff mit dem flexiblen Einsatz (3) ist;
einen inneren Spritzenzylinder (5) mit einem vorderen Ende (51), einem hinteren Ende (52) und einem Hohlkörper (50) zwischen dem vorderen Ende (51) und dem hinteren Ende (52), wobei das vordere Ende (51) des inneren Spritzenzylinders (5) eine vordere Öffnung (510) umfasst, der Innendurchmesser der vorderen Öffnung (510) größer ist als der Außendurchmesser des Nadelsitzes (60), und der innere Spritzenzylinder (5) durch die hintere Öffnung (220) des äußeren Spritzenzylinders (2) in den äußeren Spritzenzylinder (2) eingeführt werden kann;
ein flexibles Dichtungselement (8), das an der Innenwand des vorderen Endes (51) des inneren Spritzenzylinders (5) angebracht ist, damit der Hohlkörper (50) des inneren Spritzenzylinders (5) einen abgedichteten Raum bildet;
**dadurch gekennzeichnet, dass**:
eine Antidruckstruktur (7) mit dem flexiblen Dichtungselement (8) verbunden ist, um zu verhindern, dass das flexible Dichtungselement (8) während einer Injektion in den Hohlkörper (50) des inneren Spritzenzylinders (5) rutscht;
wodurch, wenn der innere Spritzenzylinder (5) in Richtung der vorderen Öffnung (210) des äußeren Spritzenzylinders (2) bewegt wird, die Antidruckstruktur (7) mit der Nadeleinheit (6) verbunden wird, wobei das vordere Ende (51) des inneren Spritzenzylinders (5) gegen den flexiblen Einsatz (3) drückt, um die Kombination aus Nadeleinheit (6) und flexiblem Einsatz (3) aus dem gegenseitigen Eingriff zu lösen, wonach das flexible Dichtungselement (8) durch die von der Nadeleinheit (6) aufgebrachte Gegenkraft in den Hohlkörper (50) des inneren Spritzenzylinders (5) gedrückt wird.

2. Sicherheitsspritze nach Anspruch 1, wobei sich der abgedichtete Raum im Hohlkörper (50) des inneren Spritzenzylinders (5) in einem Niederdruckzustand befindet.

3. Sicherheitsspritze nach Anspruch 1 oder 2, wobei die Antidruckstruktur (7) Folgendes umfasst:
ein Kopfteil (72), das in der Lage ist, sich mit dem flexiblen Dichtungselement (8) zu verbinden;
ein Halsteil (73), das mit dem Kopfteil (72) verbunden ist, wobei das Halsteil (73) am vorderen Ende (51) des inneren Spritzenzylinders (5) einrastet, um zu verhindern, dass das flexible Dichtungselement (8) in den Hohlkörper (50) des inneren Spritzenzylinders (5) rutscht; und
ein Verbindungsteil (71), das mit dem Halsteil (73) verbunden ist, wobei das Verbindungsteil (71) in der Lage ist, sich mit dem Nadelsitz (60) der Nadeleinheit (6) zu verbinden.

4. Sicherheitsspritze nach Anspruch 3, wobei die Antidruckstruktur (7) zumindest ein Trennteil (74) umfasst, das aus dem Halsteil (73) und dem Verbindungsteil (71) besteht.

5. Sicherheitsspritze nach Anspruch 4, wobei der Nadelsitz (60) der Nadeleinheit (6) ein Kombinationsteil umfasst und wobei, wenn der innere Spritzenzylinder (5) in Richtung der vorderen Öffnung (210) des äußeren Spritzenzylinders (2) bewegt wird, das Trennteil (74) der Antidruckstruktur (7) nach innen gezogen wird und der Außenrand des Halsteils (73) der Antidruckstruktur (7) innerhalb der vorderen Öffnung (510) des inneren Spritzenzylinders (5) verkleinert wird, wobei das Verbindungsteil (71) des Trennteils (74) mit dem Kombinationsteil der Nadeleinheit (6) verbunden wird, sodass die Antidruckstruktur (7) mit der Nadeleinheit (6) in verbunden wird.

6. Sicherheitsspritze nach Anspruch 1 oder 2, wobei der Nadelsitz (60) der Nadeleinheit (6) ein Einsetzteil (601) umfasst, das mit dem Nadelkörper (62) verbunden ist, und der Nadelkörper (62) von diesem Einsetzteil (601) des Nadelsitzes (60) abgetrennt werden kann.

7. Sicherheitsspritze nach Anspruch 1 oder 2, weiters einen Verbindungssitz (214') umfassend, der angrenzend an das vordere Ende (21) des äußeren Spritzenzylinders (2) angeordnet ist, wobei der Verbindungssitz (214') einen Durchlass umfasst, der mit der vorderen Öffnung (210) des äußeren Spritzenzylinders (2) kommuniziert, wobei der Nadelsitz (60), der mit dem flexiblen Einsatz (3) verbunden ist, vom Verbindungssitz getragen wird, wodurch der Nadelsitz (60) in einem vorbestimmten Abstand von der vorderen Öffnung (210) des äußeren Spritzenzylinders (2) gehalten wird und der Nadelkörper (62) durch den Durchlass des Verbindungssitzes und die vordere Öffnung (210) des äußeren Spritzenzylinders (2) geführt wird.

8. Sicherheitsspritze nach Anspruch 6, weiters einen Verbindungssitz umfassend, der angrenzend an das vordere Ende (21) des äußeren Spritzenzylinders (2) angeordnet ist, wobei der Verbindungssitz (214') einen Durchlass umfasst, der mit der vorderen Öffnung (210) des äußeren Spritzenzylinders (2) kommuniziert, wobei der Nadelsitz (60) mit dem flexiblen Einsatz (3) vom Verbindungssitz (214') getragen wird, wodurch der Nadelsitz (60) in einem vorbestimmten Abstand von der vorderen Öffnung (210) des äußeren Spritzenzylinders (2) gehalten wird und der Nadelkörper (62) durch den Durchlass des Verbindungssitzes (214') geführt und mit dem Einsetzteil (601) des Nadelsitzes (60) verbunden wird.

9. Sicherheitsspritze nach Anspruch 8, wobei der Verbindungssitz (214') eine Innenwandfläche umfasst, sich eine Spur (2141) auf der Innenwandfläche des Verbindungssitzes (214') befindet, sich eine Spurbahn (2142) am Ende der Spur (2141) entlang der Innenwandfläche des Verbindungssitzes (214') befindet, und sich ein Verriegelungsteil (2143) am Ende der Spurbahn (2142) befindet.

10. Sicherheitsspritze nach Anspruch 9, wobei der Nadelkörper 62 eine Einhakstruktur (623) umfasst, die mit der Spur (2141) übereinstimmt, wobei der Nadelkörper (62) durch den Durchlass (212) entlang der Spur (2141) geführt wird, und die Einhakstruktur (623) des Nadelkörpers (62) mithilfe des Verriegelungsteils (2143) entlang der Spurbahn (2142) verriegelt wird, sodass der Nadelkörper (62) mit dem Einsetzteil (601) des Nadelsitzes (60) verbunden ist.

## Revendications

1. Seringue de sécurité, comprenant:
un fût de seringue extérieur (2) ayant une ouverture (210) à l'extrémité avant et une ouverture (220) à l'extrémité arrière;
un siège flexible (3) monté sur une paroi intérieure dudit fût de seringue extérieur (2);
une unité d'aiguille (6) comprenant un siège d'aiguille (60) et un corps d'aiguille (62) relié audit siège d'aiguille (60), où ledit siège d'aiguille (60) est amoviblement en prise avec ledit siège flexible (3);
un fût de seringue intérieur (5) ayant une extrémité avant (51), une extrémité arrière (52) et un corps creux (50) entre ladite extrémité avant (51) et ladite extrémité arrière (52), où ladite extrémité avant (51) dudit fût de seringue intérieur (5) comprend une ouverture (510) à l'extrémité avant, un diamètre intérieur de ladite ouverture (510) à l'extrémité avant étant plus grand qu'un diamètre extérieur dudit siège d'aiguille (60), et ledit fût de seringue intérieur (5) peut être inséré dans ledit fût de seringue extérieur (2) à travers ladite ouverture (220) à l'extrémité arrière dudit fût de seringue extérieur (2);
un élément d'étanchéité flexible (8) verrouillé contre une paroi intérieure de ladite extrémité avant (51) dudit fût de seringue intérieur (5) pour amener ledit corps creux (50) dudit fût de seringue intérieur (5) à être un espace étanche;
**caractérisé par**:
une structure anti-compression (7) combinée avec ledit élément d'étanchéité flexible (8) pour empêcher que ledit élément d'étanchéité flexible (8) glisse dans ledit corps creux (50) dudit fût de seringue intérieur (5) durant l'injection;
par quoi, pendant que ledit fût de seringue intérieur (5) est amené vers ladite ouverture (210) à l'extrémité avant dudit fût de seringue extérieur (2), ladite structure anti-compression (7) est combinée avec ladite unité d'aiguille (6), ladite extrémité avant (51) dudit fût de seringue intérieur (5) est contre ledit siège flexible (3) pour libérer la combinaison de ladite unité d'aiguille (6) et dudit siège flexible (3), et ensuite ledit élément d'étanchéité flexible (8) est poussé dans ledit corps creux (50) dudit fût de seringue intérieur (5) par une force de réaction appliquée par ladite unité d'aiguille (6).

2. Seringue de sécurité selon la revendication 1, dans laquelle ledit espace étanche dans ledit corps creux (50) dudit fût de seringue intérieur (5) est dans un état de basse pression.

3. Seringue de sécurité selon la revendication 1 ou 2, dans laquelle ladite structure anti-compression (7) comprend:
une partie de tête (72) apte à être combinée avec ledit élément d'étanchéité flexible (8),
une partie de col (73) reliée à ladite partie de tête (72), ladite partie de col (73) est verrouillée à ladite extrémité avant (51) dudit fût de seringue intérieur (5) pour empêcher le glissement dudit élément d'étanchéité flexible (8) dans ledit corps creux (50) dudit fût de seringue intérieur (5); et
une partie de connection (71) reliée à ladite partie de col (73), ladite partie de connection (71) est apte à être combinée avec ledit siège d'aiguille (60) de ladite unité d'aiguille (6).

4. Seringue de sécurité selon la revendication 3, dans laquelle la structure anti-compression (7) comprend au moins une partie de séparation (74) formée par ladite partie de col (73) et ladite partie de connection (71).

5. Seringue de sécurité selon la revendication 4, dans laquelle ledit siège d'aiguille (60) de ladite unité d'aiguille (6) comprend une partie de combinaison, pendant que ledit fût de seringue intérieur (5) est amené vers ladite ouverture (210) à l'extrémité avant dudit fût de seringue extérieur (2), ladite partie de séparation (74) de ladite structure anti-compression (7) est tirée vers l'intérieur, et un bord extérieur de ladite partie de col (73) de ladite structure anti-compression (7) est réduit dans ladite ouverture (510) à l'extrémité avant dudit fût de seringue intérieur (5), ladite partie de connection (71) de ladite partie de séparation (74) est combinée avec ladite partie de combinaison de ladite unité d'aiguille (6) et ainsi ladite structure anti-compression (7) est combinée avec ladite unité d'aiguille (6).

6. Seringue de sécurité selon la revendication 1 ou 2, dans laquelle ledit siège d'aiguille (60) de ladite unité d'aiguille (6) comprend une partie de jonction (601) à combiner avec ledit corps d'aiguille (62), et ledit corps d'aiguille (62) peut être séparé de ladite partie de jonction (601) dudit siège d'aiguille (60).

7. Seringue de sécurité selon la revendication 1 ou 2, comprenant en outre un siège combiné (214') disposé d'une manière adjacente à une extrémité avant (21) dudit fût de seringue extérieur (2), ledit siège combiné (214') comprenant un passage traversant communiquant avec ladite ouverture (210) à l'extrémité avant dudit fût de seringue extérieur (2), ledit siège d'aiguille (60) en prise avec ledit siège flexible (3) est retenu par ledit siège combiné, ainsi ledit siège d'aiguille (60) est maintenu à une distance prédéterminée de ladite ouverture (210) à l'extrémité avant dudit fût de seringue extérieur (2), et ledit corps d'aiguille (62) est passé à travers ledit passage traversant dudit siège combiné et de ladite ouverture (210) à l'extrémité avant dudit fût de seringue extérieur (2).

8. Seringue de sécurité selon la revendication 6, comprenant en outre un siège combiné disposé d'une manière adjacente à une extrémité avant (21) dudit fût de seringue extérieur (2), ledit siège combiné (214') comprenant un passage traversant communiquant avec ladite ouverture (210) à l'extrémité avant dudit fût de seringue extérieur (2), ledit siège d'aiguille (60) en prise avec ledit siège flexible (3) est retenu par ledit siège combiné (214'), ainsi ledit siège d'aiguille (60) est maintenu à une distance prédéterminée de ladite ouverture (210) à l'extrémité avant dudit fût de seringue extérieur (2), et ledit corps d'aiguille (62) est passé à travers ledit passage traversant dudit siège combiné (214') et est combiné avec ladite partie de jonction (601) dudit siège d'aiguille (60).

9. Seringue de sécurité selon la revendication 8, dans laquelle ledit siège combiné (214') comprend une surface de paroi intérieure, un chemin (2141) se situe dans ladite surface de paroi intérieure dudit siège combiné (214'), une voie de chemin (2142) se trouve dans l'extrémité dudit chemin (2141) le long de ladite surface de paroi intérieure dudit siège combiné (214'), et une partie de verrouillage (2143) se trouve dans l'extrémité de ladite voie de chemin (2142).

10. Seringue de sécurité selon la revendication 9, dans laquelle ledit corps d'aiguille (62) comprend une structure de crochet (623) correspondant audit chemin (2141), ledit corps d'aiguille (62) est passé à travers ledit passage traversant (212) le long dudit chemin (2141), et ladite structure de crochet (625) dudit corps d'aiguille (62) est verrouillée avec ladite partie de verrouillage (2143) le long de ladite voie de chemin (2142), ainsi ledit corps d'aiguille (62) est combiné avec ladite partie de jonction (601) dudit siège d'aiguille (60).
